# EUROPÄISCHE PATENTSCHRIFT

(11) **EP 1 395 270 B1**
(45) Veröffentlichungstag und Bekanntmachung des Hinweises auf die Patenterteilung: **23.07.2014**
(21) Anmeldenummer: 02730214.0
(22) Anmeldetag: 26.04.2002
(51) Int. Cl.: A61K 36/185

(54) **VERWENDUNG VON PHYLLANTHUSBESTANDTEILEN ZUR HERSTELLUNG EINES ARZNEIMITTELS ZUR PRÄVENTION ODER BEHANDLUNG VON INFEKTEN DURCH RESISTENTE HEPATITIS B-VIREN**
USE OF PHYLLANTHUS COMPONENTS FOR PREPARATION OF A MEDICAMENT FOR THE PREVENTION OR TREATMENT OF INFECTIONS CAUSED BY RESISTANT HEPATITIS B VIRUS
UTILISATION D'ÉLÉMENTS DE PHYLLANTHUS POUR LA PRÉPARATION D'UN MÉDICAMENT POUR LA PRÉVENTION OU LE TRAITEMENT D'UNE INFECTION PAR LE VIRUS DE L'HEPATITE B RÉSISTANT

(30) Priorität: 26.04.2001 DE 10120627
(43) Veröffentlichungstag der Anmeldung: 10.03.2004
(73) Patentinhaber: Phytrix AG, 80686 München (DE)
(72) Erfinder: OTT, Michael, Medizinische Hochschule Hannover, 30655 Hannover (DE); MANNS, Michael, P., 30916 Isernhagen HB (DE)
(74) Vertreter: Vossius & Partner
(86) Internationale Anmeldenummer: PCT/EP2002/004641
(87) Internationale Veröffentlichungsnummer: WO 2002/087600

(56) Entgegenhaltungen:
- EP-A- 0 199 429
- WO-A-00/56347
- WO-A-98/07437
- WO-A2-00/61161
- DATABASE BIOSIS [Online] BIOSCIENCES INFORMATION SERVICE, PHILADELPHIA, PA, US; 1991 UNANDER D W: "CALLUS INDUCTION IN PHYLLANTHUS-SPP AND INHIBITION OF VIRAL DNA POLYMERASE AND REVERSE TRANSCRIPTASE BY CALLUS EXTRACTS" Database accession no. PREV199293044149 XP002210116 & PLANT CELL REPORTS, Bd. 10, Nr. 9, 1991, Seiten 461-466, ISSN: 0721-7714
- DATABASE BIOSIS [Online] BIOSCIENCES INFORMATION SERVICE, PHILADELPHIA, PA, US; März 2001 (2001-03) WANG XIN-HUA ET AL: "A comparative study of Phyllanthus amarus compound and interferon in the treatment of chronic viral hepatitis B." Database accession no. PREV200100422413 XP002210117 & SOUTHEAST ASIAN JOURNAL OF TROPICAL MEDICINE AND PUBLIC HEALTH, Bd. 32, Nr. 1, März 2001 (2001-03), Seiten 140-142, ISSN: 0125-1562
- DATABASE BIOSIS [Online] BIOSCIENCES INFORMATION SERVICE, PHILADELPHIA, PA, US; 1995 MEIXIA WANG ET AL: "Herbs of the genus Phyllanthus in the treatment of chronic hepatitis B: Observations with three preparations from different geographic sites." Database accession no. PREV199698559653 XP002151738 & JOURNAL OF LABORATORY AND CLINICAL MEDICINE, Bd. 126, Nr. 4, 1995, Seiten 350-352, ISSN: 0022-2143
- MEHROTRA R ET AL: "IN VITRO EFFECT OF PHYLLANTHUS AMARUS ON HEPATITIS B VIRUS" INDIAN JOURNAL OF MEDICAL RESEARCH, INDIAN COUNCIL OF MEDICAL RESEARCH, NEW DEHLI, IN, Bd. 93, März 1991 (1991-03), Seiten 71-73, XP000929883 ISSN: 0019-5340
- COLACINO J M ET AL: "THE IDENTIFICATION AND DEVELOPMENT OF ANTIVIRAL AGENTS FOR THE TREATMENT OF CHRONIC HEPATITIS B VIRUS INFECTION", PROGRESS IN DRUG RESEARCH - FORTSCHRITTE DERARZNEIMITTELFORSCHUNG - PROGRES DES RECHERCHES PHARMACEUTIQUES, BIRKHAEUSER VERLAG, BASEL, CH, vol. 50, 1 January 1998 (1998-01-01), pages 259-322, XP000885900, ISSN: 0071-786X
- AGYARE C ET AL: "Ellagitannins from Phyllanthus muellerianus (Kuntze) Exell.: Geraniin and furosin stimulate cellular activity, differentiation and collagen synthesis of human skin keratinocytes and dermal fibroblasts", INTERNET CITATION, [Online] 15 May 2011 (2011-05-15), pages 1-14, XP007922299, Retrieved from the Internet: URL:http://www.ncbi.nlm.nih.gov/pubmed/210 36574> [retrieved on 2013-01-01]

## Beschreibung

Die Erfindung betrifft die in den Ansprüchen charakterisierten Ausführungsformen.

Insbesondere betrifft die Erfindung die in den Ansprüchen definierte Verwendung von aus einem oder mehreren Phyllanthusbestandteil(en) durch Extraktion gewonnenen Stoffgemischen zur Herstellung eines Arzneimittels zur Prävention oder Behandlung von Infektionskrankheiten, die durch ein Hepatitis B Virus, das resistent gegen Nucleosid Analoga ist, hervorgerufen werden oder an deren Entwicklung oder Progression ein Hepatitis B Virus, das resistent gegen Nucleosid Analoga ist, beteiligt ist. Des weiteren betrifft die Erfindung die in den Ansprüchen definierte Verwendung von aus einem oder mehreren Phyllanthusbestandteil(en) durch Extraktion gewonnenen Stoffgemischen zur Inhibierung der Vermehrung eines Hepatitis B Virus, das resistent gegen Nucleosid Analoga ist.

Ferner betrifft die Erfindung die in den Ansprüchen definierten Verfahren zur Inhibierung der Vermehrung eines Hepatitis B Virus, das resistent gegen Nucleosid Analoga ist, wobei man aus einem oder mehreren Phyllanthusbestandteil (en) gewonnene Stoffgemische mit den Viren in Kontakt bringt. In einer bevorzugten Ausführungsform der Erfindung sind die Nucleosid Analoga ausgewählt aus der Gruppe bestehend aus Lamivudine und Famciclovir.

Die Pflanzengattung Phyllanthus gehört zur Unterfamilie der Phyllanthoideae, die ihrerseits zur Familie der Euphorbiaceae gerechnet wird. Die Gattung Phyllanthus umfaßt insgesamt ca. 700 Arten, die in den tropischen und subtropischen Gebieten in Australien, China, den Phillipinen, Thailand, Indonesion, Burma, Indien, Ost- und Westafrika sowie Nordamerika, Mexiko, Kuba, der Karibik und Venezuela beheimatet sind. Nur sehr selten werden Vertreter der Gattung Phyllanthus in den nördlichen gemäßigten Zonen angetroffen.

Aufgrund der Heterogenität der Gattung Phyllanthus sind nur wenige gattungsspezifische Merkmale bekannt. Bei den meisten Vertretern der Gattung Phyllanthus handelt es sich um einhäusige (monözische) oder zweihäusige (diözische) Kräuter, Sträucher oder Bäume mit vielfältigem Wuchs (Habitus), die Stengel oder Äste, welche in zwei bis drei unterschiedliche Typen differenziert sind, aufweisen. So besitzt Phyllanthus sowohl aufrechte lange Triebe von unbegrenztem Wuchs als auch kurze Triebe und horizontale blättrige oder blütige kurze Triebe von begrenztem Wuchs. Häufig werden zwei unterschiedliche Typen an Blättern beobachtet, wobei die Niederblätter an den aufrechten und kurzen Trieben und die Laubblätter an den horizontalen Trieben anzutreffen sind. Die Blüten sind generell klein und achselständig, wobei die weiblichen Blüten einschließlich ihrer Blütenstiele und Kelchblätter robuster gebaut sind als die männlichen Blüten. Da die Gattungsgrenzen zwischen den einzelnen Gattungen nicht scharf sind und einige Phyllanthusarten nach Auffassung verschiedenen Gattungen zugeordnet werden können, ist ein klares Bild über hinreichend gattungsspezifische Inhaltsstoffe bzw. Gruppen von Inhaltsstoffen nur schwer zu gewinnen. Einheitlich ist lediglich das Fehlen von Milchsaft, was als Familienmerkmal der Unterfamilie Phyllanthoideae gilt. Einige charakteristische Stoffgruppen treten jedoch in mehr oder weniger großer Verbreitung in Phyllanthus auf. Hierbei sind zunächst die Alkaloide, insbesondere die Alkaloide des vorherrschenden Securinin-Typs zu nennen, die nur in einigen Arten vorkommen. Alkaloide wurden beispielsweise in allen Pflanzenteilen von Phyllanthus niruri nachgewiesen. Weiterhin treten Verbindungen, die zu den cyanogenen Glykosiden gezählt werden, vereinzelt auf. Sie zählen zu den von Tyrosin hergeleiteten Abkömmlingen der Klasse. Ein typischer Vertreter hierfür ist Taxiphyllin und 2-Hydroxy-2-(4-Hydroxyphenyl)acetonitril in den Blättern von Phlyllanthus gasstroemii. Gerbstoffe, vor allem Gallotannine und Ellagitmnine sowie deren Vorstufen kommen bevorzugt auf holzigen Arten in den Tropen vor. Hauptvertreter hierbei sind Phyllanthus emblica mit Phyllemblin (Ethylgallat) in den Früchten. Weiterhin ist Phyllanthusin, ein Ellagitannin-Derivat im Kraut von Phyllanthus amarus beschrieben. Eine weitere große Gruppe der Inhaltsstoffe betrifft die Lignane, wobei im Kraut von Phyllanthus amarus und in allen Teilen von Phyllanthus niruri Lignane wie beispielsweise Phyllanthin und Cyclolignane oder Tetralignane wie beispielsweise Hypophyllanthin vorkommen. Außerdem sind Sesquiterpene in Phyllanthus acuminatus beschrieben, die chemisch zu den Epoxiden von tricyclischen Sesquiterpenestern mit Spiranstruktur und teilweise esterartig gebundenen Biosen gehören. Daneben kommen Triterpene in allen Pflanzenteilen vergleichsweise häufig vor. Neben Phytosteroirm (Sitosterol), Abkömmlingen vom Friedelan-, Olean- und Lupan-Typ (Betulin und Glochidonol in Phyllanthus reticulatus) ist das Auftreten von Verbindung des Euphan-Typs auffällig, wie z.B. Phyllanthenol in Phyllanthus niruri.

Insbesondere Phyllanthus amarus und Phyllanthus niruri finden in der Volksmedizin z.B. in Nigeria, Kuba oder in Jamaika Anwendung bei Fieber (Unander et al. 1991 J Ethno Pharmacol 34: 97-133). Daneben wird das Kraut einiger Phyllanthusarten bei Diarrhoe, zur Steigerung der Harnmenge, als Abführmittel sowie bei Spasmen und Koliken angewandt. Beschrieben sind ferner die Wirksamkeit von Phyllanthus bei Diabetes (Unander a.a.O.), wobei jedoch die Wirksamkeit bei diesen Anwendungen wissenschaftlich nicht belegt ist.

WO 98/07437 beschreibt, dass von 550 bekannten Spezies der Gattung Phyllanthus sieben Spezies in der Behandlung von HBV/Gelbsucht medizinische Aktivität aufweisen.

WO 00/61161 gibt an, dass die Gattung ca. 700 Spezies umfasst, wobei 24 Spezies Aktivität gegen Hepatitis zeigen.

Mehrotra et al. (Indian Journal of Medical Research, Indian Council of Medical Research, New Delhi, IN, Bd. 93, März 1991 (1991-03), Seiten 71-73) beschreibt die Aktivität von P. amarus Extrakten gegenüber HBV-Antigenen durch Inhibition der Interaktion zwischen HBsAg/HBeAg und den entsprechenden Antikörpern.

EP 0 199 426 beschreibt ebenfalls die *in vitro* antivirale Aktivität von Phyllanthus Extrakt gegenüber HBV basierend auf HBsAg-Bindeaktivität und Inhibierung der DNA Polymerase.

In wissenschaftlichen Untersuchungen konnte gezeigt werden, daß eine Reihe von hydrolisierbaren Gerbstoffen der Gattung Phyllanthus wichtige eukaryotische Proteinkinasen wie die cAMP-abhängige Proteinkinase der Rattenleber, die CA²⁺-abhängige Proteinkinase des Weizenembryos, die Ca²⁺- und phospholipidabhängige Proteinkinase C (PKC) des Rattenhirns inhibieren, wobei die Gerbstoffe Amariin, Geraniin und das Phenazin-Derivat des Geraniins jeweils am stärksten wirksam sind. Wissenschaftlich belegt ist außerdem eine antimikrobielle Wirkung eines Trockenextraktes von Phyllanthus amarus, der mit 95% Ethanol drei Stunden unter Rückfluß aus der Pflanze extrahiert wurde und das Wachstum von Bacillus subtilis und Staphylococcus aureus inhibierte. Dabei zeigte sich in einem Agarplatten-Diffusionstest eine Gabe von 0,1 ml dieses wäßrigen Extraktes in einer Konzentration von 50 mg/ml eine mäßige Hemmwirkung (Hemmzone < 15 mm) eine Positivkontrolle von 0,1 ml Streptomycin in einer Konzentration von 10 mg/ml bewirkte eine Hemmzone von 35 mm. Im gleichen Testsystem war der Extrakt gegen Pseudomonas aeruginosa, Escherichia coli, Aspergillus niger und Candida albicans unwirksam. Dagegen zeigte ein Trockenextrakt der mit Methanol bei 20°C extrahierten Ganzpflanze eine Wirkung gegen Staphylococcus aureus, aber nicht gegen E. coli, A. niger und C. albicans.

Unter den humanpathogenen Viren mit einem doppelsträngigen DNA-Genom sind insbesondere fünf verschiedene Virusfamilien zu nennen: Hepadnaviridae, Papovaviridae, Adenoviridae, Herpesviridae und Poxviridae. In nahezu allen Familien (Ausnahme Poxviridae) befinden sich Vertreter, die im Menschen persistierende Infektionen hervorrufen können. Dabei stehen Herpes-, Papova- und Hepadnaviren in enger Beziehung zu Tumorerkrankungen des Menschen, was darauf schließen läßt, daß Doppelstrang-DNA-Viren über Möglichkeiten verfügen, die Regulation der Zellteilung und des Zelltodes zu beeinflussen.

Die Familie Hepadnaviridae, deren Prototyp das Hepatitis B Virus (HBV) ist, steht entwicklungsgeschichtlich den Retroviridae nahe. Viele Details ihres Replikationszyklusses lassen vermuten, daß sich beide Familien im Laufe der Evolution aus einem gemeinsamen Vorläufer entwickelt haben. Die wesentlichste Gemeinsamkeit ist, daß beide Virenfamilien in Besitz einer Reversen Transkriptase (RT) sind, die für die Replikation beider Virenfamilien essentiell ist. Dies legt gleichzeitig nahe, daß dieser essentielle Bestandteil des Virus auch ein wichtiges Angriffsziel für Therapeutika ist.

Die Übertragung des HBV erfolgt durch Blut oder Körperflüssigkeiten, dabei dient der Mensch als Erregerreservoir, jedoch ist eine experimentelle Übertragung auf Schimpansen möglich. Die Konzentration an infektiösen Partikeln in akut oder chronisch infizierten Patienten kann Größenordnungen von 10⁹/ml Blut erreichen. Das Virus kann bei infizierten Schwangeren selten vor, meist während der Geburt an das Kind weitergegeben werden. Dies ist vor allem in den hochendemischen Gebieten wie Ostasien, Zentral- und Westafrika der Fall, wo etwa 20-80 % der Bevölkerung mit HBV durchseucht sind. Zum jetzigen Zeitpunkt geht man davon aus, daß etwa 350 Millionen Menschen chronisch mit HBV infiziert sind. In Deutschland ist das Virus nur sporadisch-endemisch verbreitet, nur circa 0,5 % der Bevölkerung sind HbsAg-positiv und etwa 5 % besitzen Antikörper.

Das Zietorgan des HBV ist die Leber. Bei Befall kann es nach einer etwa 2-6 monatigen Inkubationszeit zu einer akuten Hepatitis kommen. In 65 % der Fälle verläuft die Infektion symptomlos, während in 35 % der Fälle die Patienten eine Leberentzündung ausbilden. Dabei ist das Hauptsymptom einer akuten Hepatitis die Ausprägung einer Gelbsucht (Ikterus), einhergehend mit der Vergrößerung der Leber. Daneben werden aplastische Anämien und Exantheme beobachtet. Eine Ausheilung der Hepatitis erfolgt meist nach 4-6 Wochen. Auch nach Besserung des akuten Zustandes kann die Leberentzündung in eine chronische Verlaufsform übergehen. Etwa 5-10 % aller HBV-Infektionen bei Erwachsenen nehmen diesen chronischen Verlauf. Erfolgt die Infektion prä- oder perinatal, nehmen jedoch bis zu 90 % einen chronischen Verlauf, was insbesondere in den endemischen Regionen ein massives Problem darstellt. 60 % dieser chronisch infizierten Patienten bleiben asymptomatische Virusträger. Die symptomatischen, chronischen Infektionen werden in zwei Formen unterteilt. Man unterscheidet eine chronisch-aggressiv-progrediente Hepatitis (CAH) von der chronisch-persistenten Hepatitis (CPH), wobei die CPH in die CAH übergehen kann. Folgen einer chronischen Erkrankung sind die Leberzirrhose und das hepatozelluläre Karzinom (HCC).

Pro Jahr sterben etwa 250 000 Menschen an den Folgen dieses Tumors. HCC tritt dabei gehäuft bei Personen mit einer chronisch persistierenden HBV Infektion auf, wobei die Wahrscheinlichkeit, an einem HCC zu erkranken, mit ansteigender Infektionsdauer zunimmt. Ein Grund für die Entstehung eines HCC ist die Integration der viralen DNA in das Wirtsgenom. Dabei erfolgt oft die Öffnung der ringförmigen DNA im Bereich des Replikationsursprungs, so daß kein Virus aber noch das HBs- und das X-Gen exprimiert werden können, wobei insbesondere das X-Protein im Verdacht steht, transformierende Eigenschaften zu besitzen. Des weiteren kann auch die unspezifische Integration des Genoms selbst zur Zelltransformation führen. HBV wirkt synergistisch mit Aflatoxinen bei der Ausprägung eines HCC. Dabei handelt es sich um Difuran-Cumarin Derivate, die als Stoffwechselprodukt des Schimmelpilzes Aspergillus flavus sich häufig in verdorbener Nahrung anreichern. Dieses Gift hat eine hohes mutagenes Potential und kann mit dem Tumorsuppressor p53 interagieren. Bei Patienten, die hohen Aflatoxin-Konzentrationen ausgesetzt waren, fand man gehäuft Mutationen im p53-Gen. Des weiteren führen auch Koinfektionen mit HBV und HCV zu einem erhöhten Risiko, ein HCC auszubilden.

Zur Prävention einer HBV-Infektion sind im Laufe der letzten zwei Jahrzehnte verschiedene Wege eingeschlagen worden. So ist die Zahl der Infektionen in den westlichen Industrienationen durch ausgedehnte Impfschutz-Programme auf ein Minimum reduziert worden. In seltenen Fällen kann es jedoch trotz Impfung zu einer akuten und später chronisch-persistierenden Hepatitis B-Infektion kommen. Werden die ersten Symptome einer akuten HBV-Infektion erkannt, kann eine Nachimpfung oder die passive Immunisierung durch α-HBV-Immunglobulinen eine Ausbreitung verhindern.

Colacino et al. (Progress in Drug Research - Fortschritte der Arzneimittelforschung - Progres des Recherches Pharmaceutiques, Birkhaeuser Verlag, Basel, CH, Bd. 50, 1. Januar 1998 (1998-01-01), Seiten 259-322, ISSN: 00071-786X) gibt einen umfassenden Überblick über HBV-Struktur und Replikationsstrategie sowie die Verwendung von Zellkultursystemen, *in vitro* virale Polymerase-Systeme und Tiermodelle um anti-HBV Agenzien zu identifizieren und evaluieren. Insbesondere werden Nucleosid-Analoga als antivirale Mittel diskutiert.

Bei chronisch Infizierten kann die Gabe von Interferon α bei 20-30 % der Fälle das Virus aus der Leber eliminieren und bei weiteren 30 % die Zahl der Viren im Blut deutlich reduzieren. Neuerdings wurden eine Reihe von Nukleosidanaloga entdeckt, die die reverse Transkriptase des HBV hemmen. In der Klinik Eingang gefunden hat bereits Lamivudin. Weitere Nucleosid Analoga wie beispielsweise Famciclovir werden klinisch getestet. Nachteile der antiviralen Therapie sind, daß es sehr häufig zu einem Wiederaufflammen der Erkrankung nach Absetzen der Mittel kommt. Gegen die Nucleosid Analoga bilden sich zudem relativ schnell resistente Varianten. Daher erscheint diese Behandlungsmethode für eine langfristige Behandlung fraglich und im Hinblick auf die evolutionäre Selektion von HBV zu Gunsten von resistenten HBV Stämmen epidemiologisch bedenklich.

Es besteht daher großer Bedarf sowohl bestehende, durch das Hepatitis B Virus insbesondere von Stämmen, die gegen Nucleosid Analoga resistent sind, hervorgerufene Infektionen zu behandeln als auch bei Risikogruppen wie z.B. Drogenabhängigen oder Kindern infizierter Mütter der Entstehung prophylaktisch entgegen zu wirken. Der vorliegenden Erfindung liegt deshalb die Aufgabe zugrunde, Mittel und Wege bereitzustellen, um Infektionen und darauf beruhende Erkrankungen, die durch gegen Nucleosid Analoga resistente Hepatitis B Viren, ausgelöst werden, zu behandeln und deren Entstehung durch Prävention vorzubeugen. Diese Aufgabe wird durch die in den Ansprüchen gekennzeichneten Ausführungsformen gelöst.

Die Erfindung betrifft somit die in den Ansprüchen definierte Verwendung von aus einem oder mehreren Phyllanthusbestandteil(en) durch Extraktion gewonnenen Stoffgemischen zur Prävention oder Behandlung von Infektionskrankheiten, die durch ein Hepatitis B Virus, das resistent gegen Nucleosid Analoga ist, hervorgerufen werden oder an deren Entwicklung oder Progression ein Hepatitis B Virus, das resistent gegen Nucleosid Analoga ist, beteiligt ist.

Unter den Begriff "Phyllanthusbestandteil(en)", wie erfindungsgemäß verwendet, fallen alle Bestandteile der gesamten Pflanze, wie z.B. Blätter, Rinde, Blüten, Samen, Früchte, Stengel, Äste, Stamm, Wurzeln und Holz, sowie Teile hiervon, wie z.B. Blatt- oder Wurzelspitzen. Diese Phyllanthusbestandteile können dieselben, ähnliche oder nicht verwandte Inhaltsstoffe aufweisen. Daher können verschiedene Phyllanthusbestandteile einzeln oder miteinander sowie verschiedene Phyllanthusbestandteile von verschiedenen Phyllanthusarten einzeln oder miteinander verwendet werden. Unter "mehreren Phyllanthusbestandteilen ist auch die Gesamtheit der Phyllanthusbestandteile beispielsweise in Form von Ganzpflanzen oder Extrakten daraus zu verstehen. Die Phyllanthusbestandteile können nach Vorbehandlung oder ohne Vorbehandlung verwendet werden. Eine Vorbehandlung könnte beispielsweise Vorgänge wie das Trocknen von Blättern umfassen.

Unter "Stoffen oder Stoffgemischen", die aus Phyllanthusbestandteilen gewonnen werden können, sind vor allem Wirkstoffe zu verstehen. Diese können in reiner oder unreiner Form verwendet werden. Diese Wirkstoffe können einzeln oder in Kombination als Wirkstoff oder Wirkstoffgemisch verwendet werden. Hierbei können unterschiedliche Wirkstoffe in unterschiedlichen Phyllanthusbestandteilen oder denselben Phyllanthusbestandteilen vorliegen. Wirkstoffgemische können aus demselben Phyllanthusbestandteil oder aus verschiedenen Phyllanthusbestandteilen gewonnen werden. Hierbei kann die Gewinnung der Wirkstoffe aus den verschiedenen Phyllanthusbestandteilen zunächst einzeln geschehen, wobei die einzelnen Wirkstoffe anschließend zu einem Wirkstoffgemisch vereint werden oder es kann ein Wirkstoffgemisch gleichzeitig aus zuvor vereinigten Phyllanthusbestandteilen gewonnen werden. Erfindungsgemäße Stoffe oder Stoffgemische können aus derselben oder aus unterschiedlichen Vertretern einer Klasse von chemischen Verbindungen bestehen. Diese chemischen Verbindungen können in natürlicher Form, d.h. unverändert durch den Prozeß der Gewinnung oder aber verändert durch den Prozeß der Gewinnung verwendet werden. Die chemischen Verbindungen können beispielsweise zyklische oder verzweigte oder unverzweigte polare oder unpolare Kohlenwasserstoffe, Lipide, Lipidderivate, Nucleinsäuren, Nucleotide, Nucleotidederivate, Nucleoside, Nucleosidderivate, Polypeptide, Peptide, Aminosäuren oder modifizierte Aminosäuren sein. Unter Derivaten sind hierbei alle aus den genannten Verbindungen ableitbaren, durch chemische Modifikation erzielten Verbindungen zu verstehen. Beispielsweise können solche Derivate durch Substitutions-, Additions-, Veresterungs-, Verseifungs- oder Kondensationsreaktionen entstehen.

Unter "Prävention" ist im Sinne dieser Erfindung die in den Ansprüchen definierte Verwendung von aus Phyllanthusbestandteilen gewonnenen Stoffgemischen als vorbeugende Maßnahme zur Vermeidung der Infektion mit gegen Nucleosid Analoga resistenten HBV oder zur Herabsetzung des Virus Load zu verstehen. Weiterhin ist darunter im Sinne dieser Erfindung die Vermeidung des Krankheitsausbruchs oder das Erreichen eines vermindert schweren Krankheitsausbruches zu verstehen. Die zur Prävention verwendeten Phyllanthusbestandteile oder daraus gewonnene Stoffe oder Stoffgemische können sich von den zur Behandlung eingesetzten Phyllanthusbestandteilen oder daraus gewonnenen Stoffen oder Stoffgemischen unterscheiden oder identisch sein. Die Verwendung zur Prävention kann in zur Behandlung unterschiedlicher oder gleicher Dosis sowie durch unterschiedliche oder gleiche Applikation erfolgen. Die Prävention kann durch einmalige oder mehrmalige Applikation(en) erfolgen. Die zur Prävention verwendete Dosis der aus Phyllanthusbestandteilen gewonnenen Stoffgemische Sollte jedoch hierbei im wesentlichen ausreichend Schutz bieten vor Infektionskrankheiten, die durch Viren gegen Nucleosid Analoga resistente Hepatitis B Viren hervorgerufen werden oder an deren Entwicklung oder Progression diese Viren beteiligt sind. Dabei sollte die zur Prävention verwendete Dosis keine schädliche Wirkung auf den Organismus haben.

Bei der erfindungsgemäßen Verwendung von aus einem oder mehreren Phyllanthusbestandteilen gewonnenen Stoffgemischen zur Behandlung von Infektionskrankheiten können diese aus Phyllanthusbestandteilen gewonnenen Stoffgemische ähnlich wie für die Prävention beschrieben eingesetzt werden. Die für die Behandlung eingesetzte Dosis kann sowohl die Ursache der Infektionskrankheiten, nämlich die Viren bzw. deren Vermehrung im Organismus als auch die Symptome der Infektionskrankheiten bekämpfen. Generell ist zu bemerken, daß die Verwendung von natürlichen Therapeutika, wie Phyllanthusbestandteile oder daraus gewonnenen Stoffe oder Stoffgemischen vorteilhaft sowohl für die Prävention als auch die Behandlung von durch gegen Nucleosid Analoga resistente HBV-bedingte Infektionskrankheiten und sekundär dadurch hervorgerufene Erkrankungen sind, da bei natürlichen Therapeutika aufgrund einer besseren pharmazeutischen Verträglichkeit häufig weniger Nebenwirkungen, die den Organismus zusätzlich belasten, zu erwarten sind.

Für die Behandlung werden die Bestandteile bzw. Stoffe oder Stoffgemische als Arzneimittel, Medizinprodukt oder medizinischer Hilfsstoff formuliert. Zur Definition von Arzneimitteln vgl. die Beschreibung weiter unten.

Infektionskrankheiten zeichnen sich insbesondere dadurch aus, daß eine Immunantwort im Organismus, hervorgerufen durch ein Pathogen, ausgelöst wird. Ziel dieser Immunantwort ist es, das Pathogen zu bekämpfen. Hierbei können jedoch auch bedrohliche Schäden an Zellen und Geweben des betroffenen Organismus entstehen, die in diesem Fall durch das eigene Immunsystem hervorgerufen werden können. Daneben kann es jedoch bei Infektionskrankheiten auch durch Schädigungen durch das Pathogen selbst kommen, da beispielsweise in befallenen Zellen Stoffwechselprozesse oder Differenzierungsprogramme aufgelöst werden können, die schädigende Wirkung auf den Organismus haben. Ursache und Symptome der Infektionskrankheiten können identische Zellen oder Gewebe betreffen aber auch unterschiedliche. So könnte beispielsweise ein veränderter Lebermetabolismus zu einer Veränderung der Zusammensetzung der im Blut enthaltenen Proteine, insbesondere der Enzyme, führen, was wiederum die Schädigung anderer Organe, wie z.B. der Niere zur Folge haben könnte. Die erfindungsgemäße Verwendung von aus Phyllanthusbestandteilen gewonnenen Stoffgemischen betrifft daher nicht nur die Prävention oder Behandlung von Ursachen der Infektionskrankheiten, wie z.B. eine Infektion durch Viren, sondern auch die Prävention oder Behandlung von sekundär auftretenden Symptomen, die auf der Anwesenheit des Pathogens im Organismus beruhen.

Als "Pathogen" im Sinne der Erfindung ist eine gegen Nucleosid Analoga resistente Hepatitis B Virus zu verstehen. Das Hepatitis B Virus im Sinne der Erfindung umfaßt hierbei insgesamt acht verschiedene Subtypen, die sich vor allem dadurch auszeichnen, daß sie allesamt bedrohliche Infektionskrankheiten hervorrufen. Von besonderer Bedeutung ist hierbei die Hepatitis, wobei es zu für den Organismus lebensbedrohlichen Veränderungen der Morphologie und damit einhergehend der Funktion der Leber kommt. Weiterhin sind aufgrund der Übertragungswege für diese Viren, insbesondere Speichel und Blut, prophylaktische Maßnahmen wie die erfindungsgemäße Verwendung von Phyllanthusbestandteilen oder daraus gewonnenen Stoffen oder Stoffgemischen zur Prävention angezeigt. Die erfindungsgemäße Phyllanthusbestandteile oder daraus gewonnene Stoffe oder Stoffgemische können die Infektionskrankheit, die durch gegen Nucleosid Analoga resistente Hepatitis B Viren hervorgerufen werden, lindern insbesondere durch Inhibierung der Vermehrung der Viren in den befallenen Zellen und Geweben. Dies kann beispielsweise durch die Inhibition einzelner oder verschiedener Enzyme geschehen, die den zellulären Veränderung im Zusammenhang mit der Infektionskrankheit zugrunde liegen. Die erfindungsgemäß verwendeten Phyllanthusbestandteile oder daraus gewonnene Stoffe oder Stoffgemische können jedoch zusätzlich beispielsweise als Immunmodulatoren eine Prävention oder Behandlung der Infektionskrankheiten, die durch Viren der Familie der Flaviviridae hervorgerufen werden, positiv beeinflussen.

Unter dem Begriff "Entwicklung oder Progression" ist zu verstehen, daß das gegen Nucleosid Analoga resistente Hepatitis B Viren als Pathogen entweder Ursache und damit an der Entwicklung entweder allein oder in Kombination mit weiteren Pathogenen beteiligt sein können oder aber die Progression einer bereits bestehenden Infektionskrankheit entweder allein oder in Kombination mit anderen Pathogenen fördern können.

Die erfindungsgemäße Verwendung zeigt überraschende und unerwartete positive Effekte bei der Prävention oder Behandlung von Infektionskrankheiten, die auf Infekten durch gegen Nucleosid Analoga resistente Hepatitis B Viren beruhen.

Diese positiven und überraschenden Effekte waren aus den bislang bekannten Daten über die Effekte von bekannten Therapeutika, insbesondere Nucleosid Analoga, nicht zu erwarten. Angesichts der Ausbildung von Resistenzen verschiedener Hepatitis B Virusstämme gegen die bereits zur Behandlung eingesetzten Arzneimittel, darunter Nucleosid Analoga, wie z.B. Lamivudine und Famciclovir, war eine erfolgreiche Behandlung durch aus Phyllanthusbestandteilen gewonnene Stoffgemische nicht zu erwarten. Selbst wenn man von den im Stand der Technik bekannten positiven pharmazeutischen Wirkungen gegen das Hepatitis B Virus durch Applikation von Phyllanthusbestandteilen ausgeht, so ist es in keiner Weise naheliegend, daß sich die pharmazeutische Wirkung von Phyllanthusbestandteilen hinsichtlich der ausbleibenden therapeutischen Effekte auf mutierte Hepatitis B Virusstämme, für die bereits Resistenzen gegen andere Wirkstoffe aufgezeigt werden konnten, von diesen anderen Wirkstoffen unterscheidet. Im Hinblick dieser Tatsachen sind die zunächst in vitro gewonnenen Daten bezüglich der Inhibition der Genexpression und der Virusreplikation von Lamivudine restistenten Hepatitis B Viren insbesondere des sogenannten "YMDD Hepatitis B Virus" um so erstaunlicher.

Insbesondere betrifft die Erfindung die in den Ansprüchen definierte Verwendung von aus einem oder mehreren Phyllanthusbestandteil(en) gewonnenen Stoffgemischen die Herstellung eines Arzneimittels zur Prävention oder Behandlung von Infektionskrankheiten, die durch ein Hepatitis B Virus, das resistent gegen Nucleosid Analoga ist, hervorgerufen werden oder an deren Entwicklung oder Progression des Hepatitis B Virus, das resistent gegen Nucleosid Analoga ist, beteiligt ist.

Arzneimittel sind erfindungsgemäß als Stoffe und Zubereitungen aus Stoffen definiert, die dazu bestimmt sind, durch Anwendung am oder im menschlichen Körper Krankheiten, Leiden, Körperschäden oder krankhafte Beschwerden zu heilen, zu lindern, zu verhüten oder zu erkennen.

Medizinprodukte sind erfindungsgemäß alle einzeln oder miteinander verbunden verwendeten Stoffe und Zubereitungen aus Stoffen oder andere Gegenstände, die vom Hersteller zur Anwendung für Menschen mittels ihrer Funktionen zum Zwecke der Erkennung, Verhütung, Überwachung, Behandlung oder Linderung von Krankheiten zu dienen bestimmt sind und deren bestimmungsgemäße Hauptwirkung im oder am menschlichen Körper weder durch pharmakologisch oder immunologisch wirkende Mittel noch durch Metabolismus erreicht wird, deren Wirkungsweise aber durch solche Mittel unterstützt werden kann.

Medizinische Hilfsstoffe sind erfindungsgemäß solche Stoffe, die zur Produktion (als aktive Ingredienzien) von Arzneimitteln eingesetzt werden.

Die wesentlichen Begriffe dieser Ausführungsform wurden bereits oben erklärt und definiert. Die Arzneimittelformulierung enthaltend die aus Phyllanthusbestandteilen gewonnenen Stoffgemische erfolgt gegebenenfalls in Kombination mit einem pharmazeutisch verträglichen Träger und/oder Verdünnungsmittel.

Beispiele für geeignete pharmazeutisch verträgliche Träger sind dem Fachmann bekannt und umfassen Phosphat-gepufferte Kochsalzlösungen, Wasser, Emulsionen wie z.B. ÖI/Wasser-Emulsionen, verschiedene Arten von Detergenzien, sterile Lösungen, etc. Arzneimittel, die solche Träger umfassen, können mittels bekannter konventioneller Methoden formuliert werden. Diese Arzneimittel können einem Individuum in einer geeigneten Dosis verabreicht werden, z.B. in einem Bereich von 1µg bis 100 mg pro Tag und Patient. Die Verabreichung kann auf verschiedenen Wegen erfolgen, z.B. intravenös, introperitoneal, subkutan, intramuskulär, lokal oder intradermal. Die Art der Dosierung wird vom behandelnden Arzt entsprechend den klinischen Faktoren bestimmt. Es ist dem Fachmann bekannt, daß die Art der Dosierung von verschiedenen Faktoren abhängig ist, wie z.B. der Größe, der Körperoberfläche, dem Alter, dem Geschlecht oder der allgemeinen Gesundheit des Patienten, aber auch von dem speziellen Mittel, welches verabreicht wird, der Dauer und Art der Verabreichung und von anderen Medikamenten, die möglicherweise parallel verabreicht werden.

In einer bevorzugten Ausführungsform der Erfindung ist die Infektionskrankheit Gelbsucht, Zirrhose oder Leberzellkarzinom.

Zu den charakteristischen Symptomen der genannten Erkrankungen wird auf medizinische Standartwerke wie z.B. Pschyrembel "Klinisches Wörterbuch", Walter de Gruyter, Berlin (1994, 2000), verwiesen.

Des weiteren betrifft die Erfindung die in den Ansprüchen definierte Verwendung von aus einem oder mehreren Phyllanthusbestandteil(en) gewonnenen Stoffgemischen zur Inhibierung der Vermehrung des Hepatitis B Virus, das resistent gegen Nucleosid Analoga ist.

Unter Inhibierung der Vermehrung von gegen Nucleosid Analoga resistente Hepatitis B Viren ist zu verstehen, daß die Replikation der Viren in der befallenen Wirtszelle oder das Ausknospen der Viren aus einer befallenen Wirtszelle oder beides verhindert wird. Weiterhin könnte der Befall von weiteren Wirtszellen der Viren verhindert werden und eine Vermehrung der Viren durch eine Blockade der Zielzellen (Wirtszellen) erzielt werden. Bei der Inhibition der Neubildung der Viren in einer befallenen Wirtszelle können vor allem Enzyme, die an der Neubildung der Viren beteiligt sind, inhibiert werden. Insbesondere sind dies Enzyme, die beispielsweise an der Replikation der viralen Nucleinsäuren (reversen Transkriptasen, Polymerasen und Helicasen) oder an der Prozessierung viraler Proteine (Proteasen) beteiligt sind. Die Inhibierung der Vermehrung durch Inhibierung des Ausknospens von Viren aus einer befallenen Wirtszelle könnte insbesondere durch Inhibition der für den Ausknospungsvorgang essentiellen viralen und/oder zelluläre Protein hervorgerufen werden. Die Inhibition der Vermehrung der Blockade von weiteren Wirtszellen könnte durch Inhibierung der Mechanismen, die an der Erkennung von Wirtszellen und Virus beteiligt sind oder die an der Aufnahme des Virus in die Wirtszelle beteiligt sind oder durch Inhibierung bewerkstelligt werden.

In einer bevorzugten Ausführungsform erfolgt die Inhibierung ex vivo oder in vitro.

Diese Ausführungsform ist insbesondere für die Weiterentwicklung von erfindungsgemäßen Wirkstoffen, die Analyse neuer Wirkstoffe oder zur Qualitätskontrolle derselben von Bedeutung. Insbesondere kann die Inhibierung auch außerhalb des Organismus in isolierten Zellen oder Geweben, so z.B. in Zellkultur, durchgeführt werden. Insbesondere in in vitro Systemen, z.B. in Zellkulturen, kann analysiert werden, welche Bestandteile von Phyllanthus die vorteilhaftesten Eigenschaften aufweist. Sofern kann eine Optimierung an Stoffen oder Stoffgemischen auf der Basis von in vitro Testsystemen vorgenommen werden. Vorzugsweise werden dazu Phyllanthusbestandteile nach üblichen chemischen/physikalischen Verfahren in Gruppen von Wirkstoffen oder in Einzelkomponenten aufgeteilt und die Einzelkomponenten in derartigen Testsystemen auf Inhibierung der Virusvermehrung getestet.

Das eine oder die mehreren Phyllanthusbestandteile stammen aus Phyllanthus amarus, Phyllanthus niruri, Phyllanthus emblica, Phyllanthus urinaria, Phyllanthus acidus, Phyllanthus acuminatus und Phyllanthus reticulatus oder aus mehreren dieser Phyllanthusarten.

In einer weiteren bevorzugten Ausführungsform der Erfindung weist das Hepatitis B Virus, das resistent gegen Nucleosid Analoga ist, eine oder mehrere Mutationen in der reversen Transkriptase auf.

Unter dem Begriff "eine oder mehrere Mutationen" sind im Sinne der Erfindung ein oder mehrere Mutationen in den Nucleinsäuresequenzen des Virus oder in den Aminosäuresequenzen daraus abgeleiteter Polypeptide zu verstehen. Hierbei können die Nucleinsäuren insbesondere DNA oder RNA umfassen. Die in der Nucleinsäuresequenz vorliegenden Mutationen können den Austausch einer Aminosäure in der Aminosäuresequenz der viralen Polypeptide bewirken, der für die Veränderung der Funktion von viralen Polypeptiden verantwortlich sein kann. Insbesondere kann hierbei die Funktion von Enzymen des Virus beeinflußt werden. Die dabei entstehenden Enzyme können verantwortlich sein für die ausgebildeten Resistenzen von Hepatitis B Virusstämmen gegen herkömmliche Wirkstoffe, wie z.B. Lamivudine oder Famciclovir. Mutationen auf Nucleinsäureebene können darüber hinaus natürlich auch mit der Effizienz der Transkription und/oder der Effizienz der Translation verschiedener Teile des viralen Genoms interferieren und somit die intrazelluläre Konzentration der viralen Enzyme in der befallenen Wirtszelle positiv oder negativ beeinflussen.
Unter "reverser Transkriptase" ist ein Enzym mit der Eigenschaft einer DNA- und RNA-abhängigen DNA-Polymerase zu verstehen. Sowohl Retroviren als auch Hepatitisviren sind im Besitz einer solchen reversen Transkriptase. Im Fall des Hepatitis B Virus ist die von dem viralen Genom codierte reverse Transkriptase ein multifunktionelles Enzym. Die reverse Transkriptase läßt sich in vier Domänen unterteilen, zum einen die funktionellen Domänen der Polymerase und RNase H Aktivität und zum anderen die strukturellen Domänen. Die von den reversen Transkriptasen katalysierten Reaktionen verlaufen analog zu den normalen DNA-Polymerase Reaktionen, und wie bei anderen DNA-Polymerasen ist auch hier ein Primer erforderlich. Zunächst ist die RNA-Matrize gewöhnlich ein Einzelstrang, und es wird ein einzelner, komplementärer DNA-Strang synthetisiert, so daß ein RNA-DNA-Hybrid entsteht. Häufig ist eine kurze Polydesoxyribothymidinsäure - Poly(dT) - ein geeigneter Primer, denn sie heftet sich an den Polyriboadenylsäureschwanz, den eukaryotische mRNAs für gewöhnlich tragen. Auf diese Weise kommt es zur Synthese einer DNA-Kopie (einer cDNA) der RNA.
Die cDNA-Kopie der mRNA kann in eine doppelsträngige DNA überführt werden, wenn man die ursprüngliche RNA-Matrize mit RNaseH oder durch alkalische Hydrolyse entfernt. Das einzelsträngige DNA-Produkt dient als sein eigener Primer (und als Matrize) für die Synthese des komplementären zweiten DNA-Stranges. Diese Reaktion kann entweder durch die DNA-Polymerase I oder durch die Reverse Transkriptase selbst katalysiert werden. Anscheinend bildet sich nahe des 3'-Hydroxyl-Ende des ersten Stranges eine kurze Haarnadelschleife, womit ein Primer zur Verfügung steht. Der fertige Doppelstrang trägt an einem Ende noch die Haarnadelschleife, die schließlich von einer einzelstrangspezifischen Nuclease abgespalten wird, so daß zwei vollkommen komplementäre DNA-Einzelstränge (ein cDNA-Doppelstrang) entstehen.

In einer besonders bevorzugten Ausführungsform der Erfindung umfassen diese eine oder mehreren Mutationen einen Austausch eines Isoleucins in ein Methionin an Position 552 oder eine dazu korrespondierende Position.

Der Austausch eines Isoleucins in ein Methionin an Position 552 der viralen reversen Transkriptase ist charakteristisch für die bereits beschriebene Lamuvidin resistente YMDD Hepatitis B Virus Mutante. Dieser Austausch vermittelt offensichtlich eine Resistenz des YMDD Hepatitis B Virus Stamms gegen Lamuvidin und andere Nucleosid Analoge in Zellkultur.

Unter einer zu Position 552 "korrespondierenden Position" ist im Sinne der Erfindung ein Austausch eines Isoleucins in ein Methionin in dem Aminosäuremotif YMDD zu verstehen, wobei das YMDD Aminosäuremotif nicht unbedingt an den Positionen 551 bis 554 vorliegen muß, sondern beispielsweise durch mögliche Deletionen oder Additionen von Nucleotiden auf Nucleinsäureebene verschoben sein kann.

In einer anderen bevorzugten Ausführungsform der Erfindung umfaßt die Prävention oder Behandlung die Inhibition von einem oder mehreren viralen Enzymen.

Unter der Inhibition von einem oder mehreren viralen Enzymen ist zu verstehen, daß die Funktion dieser Enzyme entweder direkt oder indirekt negativ beeinflußt wird. Beispielsweise kann (ohne daß die Erfinder hier oder bei den weiteren Ausführungen an eine wissenschaftliche Therapie gebunden sein wollen) eine direkte Inhibierung durch Interaktion von einer oder mehreren inhibierenden Substanzen, die in den erfindungsgemäßen Stoffgemischen enthalten sind, mit einem oder mehreren Enzymen vorliegen oder eine indirekte Inhibierung durch Interaktion dieser Inhibitoren mit Faktoren, die entweder für die Expression, die Translation oder die Prozessierung dieser Enzyme notwendig sind. Insbesondere ist hierbei die Inhibition von viralen Enzymen, wie z.B. Helicasen, Polymerasen, reversen Transkriptasen oder Proteasen zu verstehen. Eine direkte Inhibition dieser Enzyme könnte beispielsweise auf der Interaktion von in Phyllanthusbestandteilen oder daraus gewonnenen Stoffen oder Stoffgemischen enthaltenen Inhibitoren mit den katalytischen Zentrum dieser Enzyme beruhen oder mit einer Domäne, die die Aktivität reguliert. Außerdem könnten aber auch zelluläre Enzyme, die beispielsweise an der Expression, Translation oder posttranslationalen Modifikation der viralen Enzyme beteiligt sind und somit deren Funktion steuern, durch diesen Inhibitoren entweder betroffen werden oder zusätzlich betroffen werden. In jedem Fall wird aber durch die erfindungsgemäße Inhibition von einem oder mehreren viralen Enzymen die Vermehrung und/oder die aus dem Befall des Virus resultierende Infektionskrankheit bzw. deren Entwicklung oder Progression gelindert.

In einer besonders bevorzugten Ausführungsform der Erfindung umfaßt die Inhibition die Inhibition der Prozessierung von einem oder mehreren viralen Enzymen.

Unter den zuvor beschriebenen posttranslationalen Modifikationen viraler Enzyme durch erfindungsgemäße Phyllanthusbestandteile oder daraus gewonnene Stoffe oder Stoffgemische steht vor allem die Prozessierung von einem oder mehreren viralen Enzymen im Vordergrund. Unter Prozessierung ist zu verstehen, daß ein zunächst inaktives Enzym durch posttranslationale Modifikationen, beispielsweise proteolytische Spaltung, Phosphorylierung oder Glycosylierung, aktiviert wird. Durch eine erfindungsgemäße Inhibition der Prozessierung von einem oder mehreren viralen Enzymen kann daher die Funktion dieser Enzyme beeinträchtigt oder komplett inhibiert werden. Die erfindungsgemäßen Stoffgemische, die zur Inhibition der Prozessierung verwendet werden, können hierbei entweder ein oder mehrere virale Enzyme gleichzeitig inhibieren. Dies kann entweder durch Inhibition des gleichen oder aber durch Inhibition von unterschiedlichen Prozessierungsschritten, die für die Aktivierung des Enzyms benötigt werden, geschehen.

In einer weiteren besonders bevorzugten Ausführungsform ist ein Enzym die reverse Transkriptase.

Der Begriff "reverse Transkriptase" ist nach oben gegebener Definition zu verstehen.

In einer anderen besonders bevorzugten Ausführungsform weist die reverse Transkriptase einen Austausch eines Isoleucins in ein Methionin an Position 552 oder einer dazu korrespondierenden Position auf.

Auf die Bedeutung des Austausches des Isoleucins in Methionin an Position 552 wurde bereits oben hingewiesen.

In einer ferner bevorzugten Ausführungsform der Erfindung sind die Nucleosid Analoga ausgewählt aus der Gruppe bestehend aus Lamivudine und Famiciclovir.

In einer anderen bevorzugten Ausführungsform der Erfindung umfassen die Phyllanthusbestandteile die Herba-Droge, Blätter, Rinde, Blüte, Samen, Früchte, Stengel, Äste, Stamm, Wurzel und Holz.

Beschrieben sind in der Anmeldung überdies Stoffe oder Stoffgemische ausgewählt aus der Gruppe bestehend aus Alkaloiden, Gerbstoffen, Lignanen, Sesquiterpenen, Triterpenen und zyanogenen Glycosiden.

Bei Alkaloiden handelt es sich um eine Stoffgruppe, die sich dadurch auszeichnet, daß sie ein oder mehrere Stickstoff Atome enthält, meist als Heterozyklus vorliegt und im Allgemeinen salzartig an eine pflanzliche Säure gebunden ist. Chemisch können Protoalkaloide, hierunter fallen auch biogene Amine als Alkaloidvorstufen, und Pseudoalkaloide unterschieden werden. Unter Alkaloiden im engeren Sinne sind Abkömmlinge bestimmter Säurenfamilien, wie z.B. Präkursorene zu verstehen. Eine weitere wichtige Stoffklasse unter den Stoffgemischen, die aus Phyllanthusbestandteilen gewonnen werden können, stellen die zyanogenen Glycoside dar. Glycoside umfassen Verbindungen, die durch Reaktion der acetalischen Hydroxylgruppe am C1-Atom eines Zuckers mit OH-Gruppen von Alkoholen und Phenolen oder anderen Zuckern, NH-Gruppen von Aminen oder SH-Gruppen von Senfölen unter Wasseraustritt entstehen. Typische Vertreter der zyanogenen Glycoside sind Taxiphyllin und 2-hydroxy-2-(4-hydroxyphenyl)acedonitril. Gerbstoffe zeichnen sich insbesondere durch phenolische Hydroxylgruppen aus. Die Gruppe der Terpene, zu denen die Sesquiterpene und die Triterpene gehören, bestehen aus ungesättigten Kohlenwasserstoffen, die aus Isoprengrundeinheiten aufgebaut sind und als Polymer vorliegen. Die erfindungsgemäßen Stoffgemische können als Reinstoff vorliegen oder Verunreinigungen aufweisen. Weiterhin können Stoffgemische aus verschiedenen Mitgliedern dieser Gruppe bestehen und darüber hinaus zusätzlich noch weitere Stoffe oder Stoffgemische enthalten.

Beschrieben ist die Verwendung von wäßrigen unpolaren, verzweigt- oder unverzweigtkettigen Kohlenwasserstoffen mit n-Hexan bei der Extraktion des Stoffes oder Stoffgemisches.

Unpolare, verzweigt- oder unverzweigtkettige Kohlenwasserstoffe dienen insbesondere als Lösungsmittel während der Extraktion von Stoffen oder Stoffgemischen aus Phyllanthusbestandteilen, wobei sich unpolare Lösungsmittel insbesondere dadurch auszeichnen, daß sie keine polaren Gruppen enthalten. Hierunter fallen beispielsweise Benzol oder gesättigte Kohlenwasserstoffe. Insbesondere sollen sich Kohlenwasserstoffe durch ein Kohlenstoffatomgerüst von 5 bis 10 Atomen (C5- bis C10-Gerüst) auszeichnen. Unter Extraktion versteht man das selektive in Lösungbringen von Stoffen oder Stoffgemischen zu deren Gewinnung. Das Lösungsverhalten wird hierbei durch das verwendete Lösungsmittel beeinflußt.

Es können wäßrige unpolare, verzweigt- oder unverzweigtkettige Kohlenwasserstoffe mit n-Hexan bei der Extraktion des Stoffes oder Stoffgemisches verwendet werden.

Die Kohlenwasserstoffe haben ein C5- bis C10-Gerüst.

In der erfindungsgemäßen Ausführungsform werden Alkohole und/oder eine Mischung davon bei der Extraktion des Stoffgemisches verwendet wie in den Ansprüchen definiert.

Unter Alkoholen versteht man insbesondere Kohlenwasserstoffe, die eine oder mehrere OH-Gruppen an Kohlenstoffatomen besitzen. Der Extraktion der erfindungsgemäßen Stoffgemische werden Alkohole mit einem Gerüst von 1 bis 4 Kohlenstoffatomen und/oder Mischungen davon eingesetzt. Hierbei sind von besonderer Bedeutung Methanol oder Ethanol-Gemische hiervon.

In der erfindungsgemäßen Ausführungsform sind die verwendeten Alkohole kurzkettige primäre C1- bis C4-Alkohole und/oder Mischungen davon.

In einer besonders bevorzugten Ausführungsform der Erfindung sind die verwendeten Alkohole Methanol und/oder Ethanol.

In einer bevorzugten Ausführungsform werden eines oder mehrere der Phyllanthusbestandteile oder die daraus gewonnenen Stoffe oder Stoffgemische in Kombination mit anderen Wirkstoffen eingesetzt.

Die erfindungsgemäßen Stoffgemische können auch in Kombination, d.h. entweder direkt als Gemisch, parallel oder nacheinander zusammen mit anderen Wirkstoffen eingesetzt werden. Unter anderen Wirkstoffen sind vor allem die in der Therapie von Infektionskrankheiten, die durch ein Hepatitis B Virus, das gegen Nucleosid Analoga resistent ist, hervorgerufen werden, bekannten Arzneimittel zu verstehen.

In einer besonders bevorzugten Ausführungsform sind die anderen Wirkstoffe ausgewählt aus der Gruppe bestehend aus Interferon α, Interferon β, Interferon γ und Ribavirin.

Als Wirkstoffe bei der Behandlung oder Prävention von Infektionskrankheiten, die auf Mitglieder der Familie der Flaviviridae beruhen, haben sich insbesondere Interferon α, Interferon β, Interferon y und Ribavirin bewährt. Die Wirkung von Interferonen beruht hierbei vor allem auf deren modulierende Effekte auf die Zellen des Immunsystems. Eine Kombinationstherapie mit zwei oder mehr dieser Wirkstoffe sollte sich bei der Prävention wie auch bei der Behandlung des Krankheitsverlaufs besonders günstig auswirken.

In einer bevorzugten Ausführungsform der Erfindung erfolgt die Anwendung in Form einer Infusionslösung, Injektionslösung, Tablette, Salbe, Heilpackung, eines Granulats, Nahrungsergänzungsmittel, oder in Form von Klysmen.

Stoffgemische können in unterschiedlichen Formen entweder getrennt oder in Kombination angewendet werden. Insbesondere können die erfindungsgemäßen Stoffgemische in Form von Infusionslösungen oder Injektionslösungen appliziert werden. Hierbei werden aus Stoffgemischen isolierte Wirkstoffe in reiner oder unreiner Form mit einem pharmazeutisch verträglichem Lösungsmittel versetzt und in die Blutbahn eines Patienten, der an einer Infektionskrankheit, die auf der Infektion durch gegen Nucleosid Analoga resistente Hepatitis B Viren beruht, gebracht. Infusionslösungen und Injektionslösungen als Anwendungsform eignen sich daher vorzugsweise zur Behandlung. Unter Tabletten versteht man ein pulverförmiges oder granuliertes Gemisch von Wirk- und Hilfsstoffen in Form von regelmäßigen immer gleichen Formen (z.B. Diskus-, Zylinder-, Ei-, Kugel-, Stäbchen-, oder Würfelform) zur einheitlichen Dosierung. Die Wirkstoffe können hierbei in reiner oder unreiner Form aus den erfindungsgemäßen Stoffgemischen, die aus Phyllanthusbestandteilen gewonnen werden, isoliert werden. Die Hilfsstoffe dienen hierbei insbesondere zur pharmazeutischen Verträglichkeit und dazu die Wirkstoffe haltbar und lagerfähig zu machen. Eine Salbe, die einen Wirkstoff, der in reiner oder unreiner Form vorliegen kann und aus den erfindungsgemäßen Stoffgemischen hergestellt werden kann, ist eine plastisch verformbare Arzneizubereitung, die neben dem Wirkstoff eine Salbengrundlage als Basis oder Vehikel umfaßt. Heilpackungen können insbesondere entweder die erfindungsgemäßen Stoffgemische in reiner oder unreiner Form sowie die aus Stoffgemischen isolierten Wirkstoffe in reiner oder unreiner Form in Kombination mit Trägermaterialien sowie weiteren Wirkstoffen umfassen. Unter einem Granulat ist ein grobkörniges, granulahaltiges Pulvergemisch zu verstehen, das Wirkstoffe aus den erfindungsgemäßen Stoffgemischen enthält. Ein Granulat kann auch in Tablettenform vorliegen. Die erfindungsgemäßen Stoffgemische können auch als Nahrungsergänzungsmittel eingesetzt werden. Hierunter sind insbesondere neben der Verwendung von Phyllanthusbestandteilen in Form von Nahrungsmitteln auch Nahrungsergänzungsmittel z.B. in Form von Tabletten, Granulaten oder als Heiltees zu verstehen.

In einer weiteren bevorzugten Ausführungsform der Erfindung erfolgt die Anwendung oral, topisch oder parenteral.

Beschrieben sind ferner Verfahren zur Prävention oder Behandlung von Infektionskrankheiten bei einem Säuger, die durch ein Hepatitis B Virus, das resistent gegen Nucleosid Analoga ist, hervorgerufen werden oder an deren Entwicklung oder Progression ein Hepatitis B Virus, das gegen Nucleosid Analoga resistent ist, beteiligt ist, wobei dem Säuger ein oder mehrere Phyllanthusbestandteil(e) oder daraus gewonnene Stoffe oder Stoffgemische verabreicht wird.

Das Verfahren kann neben dem Schritt der Verabreichung von einem oder mehreren Phyllanthusbestandteilen oder daraus gewonnenen Stoffen oder Stoffgemischen auch weitere Schritte, wie beispielsweise die Verabreichung anderer Therapeutika oder zusätzliche therapeutische Maßnahmen umfassen. Für die erfindungsgemäßen Verfahren bzw. die in der Beschreibung derselben verwendeten Begriffe gelten ebenfalls die Definitionen gemäß der erfindungsgemäßen Verwendungen. Desweiteren umfassen die erfindungsgemäßen Verfahren auch alle Ausführungsformen der erfindungsgemäßen Verwendungen.

Der Säuger kann ein Mensch sein.

Des weiteren betrifft die Erfindung die in den Ansprüchen definierten Verfahren zur Inhibierung der Vermehrung eines Hepatitis B Virus, das gegen Nucleosid Analoga resistent ist, wobei man einen oder mehrere Phyllanthusbestandteil(e) oder daraus gewonnene Stoffe oder Stoffgemische mit den Viren in Kontakt bringt.

Die Inhibierung erfolgt ex vivo oder in vitro.

In einer besonders bevorzugten Ausführungsform der Erfindung sind die Nucleosid Analoga ausgewählt aus der Gruppe bestehend aus Lamivudine und Famciclovir.

Die für die erfindungsgemäße Verwendung beschriebenen weiteren bevorzugten oder besonders bevorzugten Ausführungsform gelten mutatis mutandis auch für die erfindungsgemäßen Verfahren.

Die Figur zeigt:

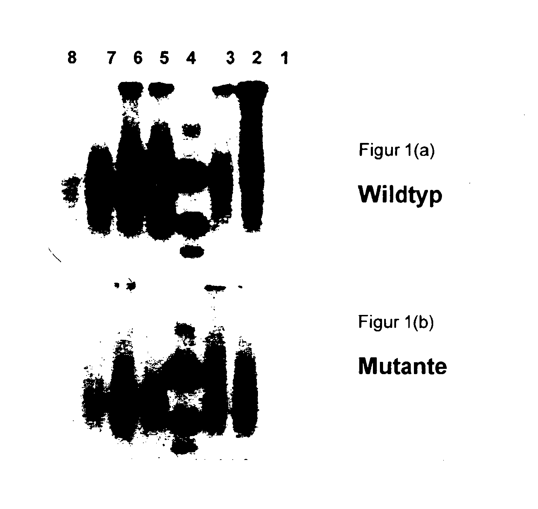

### Figur 1: In HuH7-Zellen inhibiert Phyllanthus amarus Extrakt die "de novo" Synthese von viraler DNA in Dosis-abhängiger Weise

Figur 1 (a) zeigt die Dosis-abhängige Inhibition von "Wildtyp"-HBV-DNA in HuH7-Zellen. Der Phyllanthus amarus Extrakt wurde mit Konzentrationen von 0 (negative Kontrolle) bis 1000 µg/ml getestet. Spur 1 zeigt die Negativkontrolle (es wurde kein Virus transfiziert). Spur 2 zeigt die Neusynthese von DNA (Virusnachkommen) in Abwesenheit des Phyllanthus amarus Extraktes. Spur 3 zeigt eine positive Kontrolle (im Vergleich zu Spur 2 wurde die Hälfte der HBV-DNA transfiziert). Spur 4 zeigt den Molekulargewichtsstandard (1 kb-Leiter). Spuren 5 bis 8 zeigen die Neubildung von HBV-DNA (Virusnachkommen) in Gegenwart von 100 (Spur 5), 250 (Spur 6), 500 (Spur 7) und 1000 (Spur 8) µg/ml Phyllanthus amarus.
Figur 1 (b) zeigt die Dosis-abhängige Inhibition der "YMDD (Isoleucin) Mutanten"-HBV-DNA Bildung in HuH7-Zellen. Der Phyllanthus amarus Extrakt wurde in Konzentrationen von 0 (negative Kontrolle) bis 1000 µg/ml getestet. Spur 1 zeigt die Negativkontrolle (es wurde kein Virus transfiziert). Spur 2 zeigt die Neusynthese von DNA (Virusnachkommen) in Abwesenheit des Phyllanthus amarus Extraktes. Spur 3 zeigt eine positive Kontrolle (im Vergleich zu Spur 2 wurde die Hälfte der HBV-DNA transfiziert). Spur 4 zeigt den Molekulargewichtsstandard (1 kb-Leiter). Spuren 5 bis 8 zeigen die Neubildung von HBV-DNA (Virusnachkommen) in Gegenwart von 100 (Spur 5), 200 (Spur 6), 500 (Spur 7) und 1000 (Spur 8) µg/ml Phyllanthus amarus.

Die Bespiele erläutern die Erfindung.

### Beispiel 1: In vitro Inhibition der Genexpression und der Replikation von Lamuvidin resistenten (YMDD) Hepatitis B Viren

Der inhibitorische Effekt von Phyllantus amarus Bestandteilen oder daraus gewonnenen Stoffen auf die Genexpression und die Replikation von "Wildtyp" und Lamuvidin resistenten (YMDD) Hepatitis B Viren wurde in einem Zellkulturmodell getestet, siehe auch Figur 1. In diesen Experimenten wurde ein standardisiertes Extrakt mit LAT-Nr. 01620514 auf Wasserbasis, das von Prof. Wagner, München zur Verfügung gestellt wurde, verwendet. HuH7-Zellen wurden zunächst in Dulbecco's modified Eagle Medium, das mit 10% fötalem Kälberserum, Glutamin und Penicillin Streptomycin versetzt worden war, in 5% CO₂ bei 37° C kultiviert. Eine Mycoplasmakontamination der Zellkultur wurde durch entsprechende Tests ausgeschlossen. Die Zytotoxizität des Phyllanthus amarus Extrakts wurde zunächst im WST1 Assay (Boehringer Mannheim) getestet. Eine Zytotoxizität des Extraktes konnte dadurch ausgeschlossen werden. Anschließend wurden etwa 10⁶ HuH7-Zellen ausplatiert und über Nacht kultiviert. Am nächsten Tag wurden diese Zellen mit 15 µg der Plasmid-DNA PHBV1.5 oder einem Plasmid, das das Genom der Lamuvidin resistenten YMDD Hepatitis B Virus Mutante enthält durch die Calciumphosphat-Methode transfiziert. Die Transfektionseffizienz wurde durch Co-Transfektion von 1 µg eines β-Galactosidase Expressionsplasmids durch Nachweis der β-Galactosidaseaktivtät überprüft. Die transfizierten HuH7-Zellen wurden in Medium, das 100, 250, 500 oder 1000 µg/ml des Phyllantus amarus Extrakts enthielt, für 5 Tage kultiviert. Anschließend wurden die Zellen in PBS gewaschen, gesammelt und homogenisiert. Die homogenisierte Zellsuspension wurde in einer Mikrozentrifuge bei maximaler Geschwindigkeit abzentrifugiert. Die Überstände wurden gesammelt und mit anti-HBC-Antiköpern beladenen Protein A Agarose Beads inkubiert. Nach Inkubation wurden die Beads dreimal mit TNE-Puffer gewaschen und bei 4° C für 12 Stunden darin inkubiert. Nach dem letzten Waschschritt wurden die pelletierten Beads mit DNase (1 U/ml) und RNase (1 U/µl) in TNE-Puffer in Anwesenheit von Magnesiumchlorid für 30 Minuten bei 37° C inkubiert. Nach dreimaligem Waschen des Pellets mit TNE-Puffer folgt eine Behandlung mit Proteinase K (0,5 mg/ml) in Anwesenheit von Natriumdodecylsulfat (1%) und Sacrosyl bei 37° C für 12 Stunden um die in den durch die Beads aufgereinigten Hepatitis B Partikel enthaltene virale DNA freizusetzen. Die DNA wurde anschließend Phenolchlorophorm Isoamylalkohol (25:24:1) extrahiert und in Anwesenheit von 10 µg tRNA präzipitiert. Das DNA Pellet wurde anschließend in 20 µl TE-Puffer resuspendiert und unter denaturierenden Bedinungen in einem 1%igen alkalischen Agarosegel aufgetrennt. Anschließend wurde eine Southern Analyse durchgeführt wobei die DNA auf eine Nylonmembran (Qiabrane Nylon, Fa. Qiagen, Deutschland) übertragen wurde. Zur Detektion der viralen DNA wurde monomerische DNA aus dem Plasmid pHBV1.5 isoliert und über eine "random priming procedure" mit dem Radionuklid ³²P markiert. In einer anschließenden Hybridisierung wurden 10⁶ cpm/ml dieser markierten Sonde eingesetzt. Die gewonnenen Daten zeigen, daß das Phyllanthus amarus Extrakt die "de novo" Synthese viraler DNA signifikant inhibiert in Abhängigkeit der eingesetzten Dosis des Extrakts. Die Inhibition der Denovosynthese von viraler DNA konnte bei "Wildtyp" Hepatitis B Viren bereits bei einer Konzentration von 250 µg/ml Phyllanthus amarus Extrakt beobachtet werden, bei Lamivudin-resistenten YMDD Hepatitis B Virus Mutanten bei einer Konzentration von 500-1000 mg/ml. In keinem Fall gehen die beobachteten Effekte auf Zytotoxizität des Phyllanthus amarus Extrakts zurück.

## Patentansprüche

1. Verwendung von aus einem oder mehreren Phyllanthusbestandteil(en) durch Extraktion gewonnenen Stoffgemischen zur Herstellung eines Arzneimittels zur Prävention oder Behandlung von Infektionskrankheiten, die durch ein Hepatitis B Virus, das resistent gegen Nucleosid Analoga ist hervorgerufen werden oder an deren Entwicklung oder Progression ein Hepatitis B Virus, das resistent gegen Nucleosid Analoga ist, beteiligt ist, wobei kurzkettige primäre C1- bis C4-Alkohole und/oder Mischungen davon bei der Extraktion des Stoffgemisches verwendet werden, wobei der eine oder die mehreren Phyllanthusbestandteile aus Phyllanthus amarus stammen.

2. Verwendung nach Anspruch 1, wobei die Infektionskrankheit Gelbsucht, Zirrhose oder Leberzellkarzinom ist.

3. Verwendung von aus einem oder mehreren Phyllanthusbestandteil(en) durch Extraktion gewonnenen Stoffgemischen zur *ex vivo* oder *in vitro* Inhibierung der Vermehrung des Hepatitis B Virus, das resistent gegen Nucleosid Analoga ist, wobei kurzkettige primäre C1- bis C4-Alkohole und/oder Mischungen davon bei der Extraktion des Stoffgemisches verwendet werden, wobei der eine oder die mehreren Phyllanthusbestandteile aus Phyllanthus amarus stammen.

4. Verwendung nach einem der Ansprüche 1 bis 3, wobei das Hepatitis B Virus, das resistent gegen Nucleosid Analoga ist, eine oder mehrere Mutationen in der reversen Transkriptase aufweist.

5. Verwendung nach Anspruch 4, wobei diese eine oder mehreren Mutationen einen Austausch eines Isoleucins in ein Methionin an Position 552 oder eine dazu korrespondierende Position umfassen, wobei die dazu korrespondierende Position im Aminosäuremotif YMDD liegt.

6. Verwendung nach einem der Ansprüche 1 bis 5, wobei die Prävention oder Behandlung die Inhibition von einem oder mehreren viralen Enzymen umfasst.

7. Verwendung nach Anspruch 6, wobei die Inhibition die Inhibition der Prozessierung von einem oder mehreren viralen Enzymen umfasst.

8. Verwendung nach Anspruch 6 oder 7, wobei ein Enzym die reverse Transkriptase ist.

9. Verwendung nach Anspruch 8, wobei die reverse Transkriptase einen Austausch eines Isoleucins in ein Methionin an Position 552 oder einer dazu korrespondierenden Position aufweist, wobei die dazu korrespondierende Position im Aminosäuremotif YMDD liegt.

10. Verwendung nach einem der Ansprüche 1 bis 9, wobei die Nucleosid Analoga ausgewählt sind aus der Gruppe bestehend aus Lamivudine und Famciclovir.

11. Verwendung nach einem der Ansprüche 1 bis 10, wobei die Phyllanthusbestandteile die Herba-Droge, Blätter, Rinde, Blüte, Samen, Früchte, Stengel, Äste, Stamm, Wurzel und Holz umfassen.

12. Verwendung nach einem der Ansprüche 1 bis 11, wobei die verwendeten Alkohole Methanol und/oder Ethanol sind.

13. Verwendung nach einem der Ansprüche 1 bis 12, wobei die aus einem oder mehreren Phyllanthusbestandteil(en) gewonnenen Stoffgemische in Kombination mit anderen Wirkstoffen eingesetzt werden.

14. Verwendung nach Anspruch 13, wobei die anderen Wirkstoffe ausgewählt sind aus der Gruppe bestehend aus Interferon α, Interferon β, Interferon γ und Ribavirin.

15. Verwendung nach einem der Ansprüche 1 bis 14, wobei die Anwendung in Form einer Infusionslösung, Injektionslösung, Tablette, Salbe, Heilpackung, eines Granulats, Nahrungsergänzungsmittel, oder in Form von Klysmen erfolgt.

16. Verwendung nach einem der Ansprüche 1 bis 15, wobei die Anwendung oral, topisch oder parenteral erfolgt.

17. *In vitro* oder ex *vivo* Verfahren zur Inhibierung der Vermehrung eines Hepatitis B Virus, das gegen Nucleosid Analoga resistent ist, wobei man aus einem oder mehreren Phyllanthusbestandteil(en) durch Extraktion gewonnene Stoffgemische mit den Viren in Kontakt bringt, wobei kurzkettige primäre C1-bis C4-Alkohole und/oder Mischungen davon bei der Extraktion des Stoffgemisches verwendet werden, wobei der eine oder die mehreren Phyllanthusbestandteile aus Phyllanthus amarus stammen.

18. *In vitro* oder *ex vivo* Verfahren nach Anspruch 17, wobei die Nucleosid Analoga ausgewählt sind aus der Gruppe bestehend aus Lamivudine und Famciclovir.

## Claims

1. Use of substance mixtures obtained from one or more Phyllanthus component(s) by extraction for the production of a pharmaceutical composition for prevention or treatment of infectious diseases, which are caused by a hepatitis B virus which is resistant to nucleoside analogues, or the development and progression of which involve the participation of a hepatitis B virus which is resistant to nucleoside analogues, wherein short-chain primary C1 to C4 alcohols and/or mixtures thereof are used in the extraction of the substance mixture, wherein the one or more Phyllanthus components are derived from Phyllanthus amarus.

2. Use according to claim 1, wherein the infectious disease is jaundice, cirrhosis or liver cell carcinoma.

3. Use of substance mixtures obtained from one or more Phyllanthus component(s) by extraction for *ex vivo* or *in vitro* inhibition of the propagation of the hepatitis B virus which is resistant to nucleoside analogues, wherein short-chain primary C1 to C4 alcohols and/or mixtures thereof are used in the extraction of the substance mixture, wherein the one or more Phyllanthus components are derived from Phyllanthus amarus.

4. Use according to any one of claims 1 to 3, wherein the hepatitis B virus which is resistant to nucleoside analogues has one or more mutations in the reverse transcriptase.

5. Use according to claim 4, wherein the one or more mutations comprise a substitution of an isoleucine for a methionine at position 552 or a corresponding position, wherein the corresponding position is in the amino acid motif YMDD.

6. Use according to any one of claims 1 to 5, wherein the prevention or treatment comprises the inhibition of one or more viral enzymes.

7. Use according to claim 6, wherein the inhibition comprises the inhibition of the processing of one or more viral enzymes.

8. Use according to claim 6 or 7, wherein an enzyme is the reverse transcriptase.

9. Use according to claim 8, wherein the reverse transcriptase has a substitution of an isoleucine for a methionine at position 552 or a corresponding position, wherein the corresponding position is in the amino acid motif YMDD.

10. Use according to any one of claims 1 to 9, wherein the nucleoside analogues are selected from the group consisting of lamivudine and famciclovir.

11. Use according to any one of claims 1 to 10, wherein the Phyllanthus components comprise the herba drug, leaves, bark, blossom, seeds, fruit, stem, branches, trunk, root and wood.

12. Use according to any one of claims 1 to 11, wherein the alcohols used are methanol and/or ethanol.

13. Use according to any one of claims 1 to 12, wherein the substance mixtures obtained from one or more Phyllanthus component(s) are used in combination with other active agents.

14. Use according to claim 13, wherein the other active agents are selected from the group consisting of interferon α, interferon β, interferon γ and ribavirin.

15. Use according to any one of claims 1 to 14, wherein the administration is carried out in the form of infusion solution, injection solution, tablet, ointment, medical packs, granulate, food supplement or in the form of enemas.

16. Use according to any one of claims 1 to 15, wherein the administration is oral, topical or parenteral.

17. *In vitro* or *ex vivo* method for the inhibition of the propagation of a hepatitis B virus which is resistant to nucleoside analogues, wherein the substance mixtures obtained from one or more Phyllanthus component(s) by extraction are contacted with the viruses, wherein short-chain primary C1 to C4 alcohols and/or mixtures thereof are used in the extraction of the substance mixtures, wherein the one or more Phyllanthus components are derived from Phyllanthus amarus.

18. The *in vitro* or ex *vivo* method according to claim 17, wherein the nucleoside analogues are selected from the group consisting of lamivudine and famciclovir.

## Revendications

1. Utilisation de mélanges de substances obtenus par extraction à partir d'un ou de plusieurs composants de phyllanthus pour la production d'un médicament destiné à la prévention ou au traitement des maladies infectieuses, qui sont provoquées par un virus de l'hépatite B résistant aux analogues de nucléosides, ou dans le développement ou la progression desquelles un virus de l'hépatite B résistant aux analogues de nucléosides est impliqué, des alcools primaires en C₁ à C₄ à chaîne courte et/ou des mélanges de ceux-ci étant utilisés lors de l'extraction du mélange de substances, un ou plusieurs des composants de phyllanthus provenant du Phyllanthus amarus.

2. Utilisation selon la revendication 1, dans laquelle la maladie infectieuse est la jaunisse, la cirrhose du foie ou le carcinome des cellules hépatiques.

3. Utilisation de mélanges de substances obtenus par extraction à partir d'un ou de plusieurs composants de phyllanthus pour l'inhibition *ex vivo* ou *in vitro* de la multiplication du virus de l'hépatite B résistant aux analogues de nucléosides, des alcools primaires en C₁ à C₄ à chaîne courte et/ou des mélanges de ceux-ci étant utilisés lors de l'extraction du mélange de substances, un ou plusieurs des composants de phyllanthus provenant du Phyllanthus amarus.

4. Utilisation selon l'une des revendications 1 à 3, dans laquelle le virus de l'hépatite B résistant aux analogues de nucléosides présente une ou plusieurs mutations dans la transcriptase inverse.

5. Utilisation selon la revendication 4, dans laquelle une ou plusieurs de ces mutations comprennent un remplacement d'une isoleucine par une méthionine en position 552 ou en une position qui y correspond, la position qui y correspond étant située dans le motif d'acides aminés YMDD.

6. Utilisation selon l'une des revendications 1 à 5, dans laquelle la prévention ou le traitement comprend l'inhibition d'une ou de plusieurs enzymes virales.

7. Utilisation selon la revendication 6, dans laquelle l'inhibition comprend l'inhibition de la transformation d'une ou de plusieurs enzymes virales.

8. Utilisation selon la revendication 6 ou 7, dans laquelle une enzyme est la transcriptase inverse.

9. Utilisation selon la revendication 8, dans laquelle la transcriptase inverse comprend un remplacement d'une isoleucine par une méthionine en position 552 ou en une position qui y correspond, la position qui y correspond étant située dans le motif d'acides aminés YMDD.

10. Utilisation selon l'une des revendications 1 à 9, dans laquelle les analogues de nucléosides sont choisis dans le groupe constitué par la lamivudine et le famciclovir.

11. Utilisation selon l'une des revendications 1 à 10, dans laquelle les composants de phyllanthus comprennent les drogues végétales, les feuilles, les écorces, les fleurs, les graines, les fruits, les tiges, les branches, le tronc, les racines et le bois.

12. Utilisation selon l'une des revendications 1 à 11, dans laquelle les alcools utilisés sont le méthanol et/ou l'éthanol.

13. Utilisation selon l'une des revendications 1 à 12, dans laquelle les mélanges de substances obtenus à partir d'un ou de plusieurs composants de phyllanthus sont mis en oeuvre en combinaison avec d'autres principes actifs.

14. Utilisation selon la revendication 13, dans laquelle les autres principes actifs sont choisis dans le groupe constitué par l'interféron α, l'interféron β, l'interféron γ et la ribavirine.

15. Utilisation selon l'une des revendications 1 à 14, dans laquelle l'administration s'effectue sous la forme d'une solution à perfuser, d'une solution à injecter, de comprimés, de pommades, de cataplasme, de granulés, de compléments alimentaires ou sous forme de lavements.

16. Utilisation selon l'une des revendications 1 à 15, dans laquelle l'administration s'effectue par voie orale, topique ou parentérale.

17. Procédé *in vitro* ou ex *vivo* d'inhibition de la multiplication d'un virus de l'hépatite B résistant aux analogues de nucléosides, dans lequel on met en contact des mélanges de substances obtenus par extraction à partir d'un ou de plusieurs composants de phyllanthus avec les virus, des alcools primaires en C₁ à C₄ à chaîne courte et/ou des mélanges de ceux-ci étant utilisés lors de l'extraction du mélange de substances, un ou plusieurs des composants de phyllanthus provenant du Phyllanthus amarus.

18. Procédé *in vitro* ou *ex vivo* selon la revendication 17, dans lequel les analogues de nucléosides sont choisis dans le groupe constitué par la lamivudine et le famciclovir.
